# EUROPEAN PATENT APPLICATION

(11) **EP 2 505 659 A1**
(43) Date of publication of application: **03.10.2012**
(21) Application number: 11002701.8
(22) Date of filing: 31.03.2011
(51) Int. Cl.: C12Q 1/26

(54) **Methods for the identification of dioxygenase interacting molecules and for the purification of dioxygenase proteins**

(71) Applicant: CELLZOME AG, 69117 Heidelberg (DE)
(72) Inventor: Reader, Valerie, Linton, Cambridge CB21 4HU (GB); Ramsden, Nigel, Fowlmere, Royston Herts SG8 7QP (GB)
(74) Representative: Lahrtz, Fritz

(57) **Abstract**

The present invention relates to immobilization compounds, immobilization products and preparations thereof as well as to methods and uses for the identification of dioxygenase interacting compounds or for the purification or identification of dioxygenase proteins.

## Description

The present invention relates to immobilization compounds, immobilization products and preparations thereof as well as to methods and uses for the identification of dioxygenase interacting compounds or for the purification or identification of dioxygenase proteins.

Pioneering work in the 1960s defined prolyl and lysyl hydroxylations as physiologically important oxygenase-catalyzed modification in collagen biosynthesis. More recent work on the hypoxia-sensing mechanism in animals has shown that prolyl and asparaginyl hydroxylation of the hypoxia-inducible transcription factor play central roles in sensing hypoxia (Loenarz and Schofield, 2011; Trends Biochem. Sci. 36(1): 7-18).

The cellular responses to reduced oxygen concentration (hypoxia) are mediated by the α/β-heterodimeric transcription factor hypoxia-inducible factor (HIF). Under hypoxia, HIF binds to specific DNA-sequences in promoters of genes associated with the hypoxic response, for example in angiogenesis (vascular endothelial growth factor, VEGF) or erythropoiesis (erythropoietin, EPO). The HIF-β protein is a constitutive nuclear protein, but the level of HIF-α is regulated depending on oxygen availability. HIF-α can be hydroxylated on either of two proline residues in the degradation domain of HIF-α, which target the protein to the von Hippel Lindau protein (VHL) which in turn recruits a ubiquitin ligase that mediates proteosomal destruction. In addition, the transcriptional activity of HIF is directly inhibited by hydroxylation of asparagine residue 803 in human HIF-α that blocks interaction with the transcriptional coactivator p300 (McDonough et al., 2005; J. Am. Chem. Soc. 127(21): 7680-7681).

Four hydroxylases have been identified that catalyse the hydroxylation of HIF-α: three prolyl hydroxylases (PHD1-3) and the asparaginyl hydroxylase "factor inhibiting HIF" (FIH) which belong to the family of the 2-oxoglutarate (20G)- and Fe(II)-dependent dioxygenases (20G-Fe(II) dioxygenases). The catalytic core of the proteins consists of a double-stranded β-helix (DSBH) fold containing a HX[DE] dyad (where X is any amino acid) and a conserved carboxyterminal histidine which together chelate a single iron atom. The 2OG-Fe(II) dioxygenase family comprises approximately 60 members in humans (Aravind and Koonin, 2001; Genome Biol. 2(3): RESEARCH0007-research0007.8).

Pharmacological manipulation of the HIF pathway is being pursued for the treatment of anemia, myocardial ischemia and cancer (Nagel et al, 2010; Antioxid Redox Signal. 2010 Apr;12(4):481-501).

For the development of drugs targeting dioxygenases, it is important to have assays that allow the identification and characterization of small molecule inhibitors in terms of potency and selectivity across the dioxygenase family (McDonough et al., 2005; J. Am. Chem. Soc. 127(21): 7680-7681).

Therefore, another step for the identification of selective dioxygenase inhibitors is a method that allows to determine the target selectivity of these molecules. For example, it can be intended to provide molecules that bind to and inhibit a particular drug target but do not interact with a closely related target, inhibition of which could lead to unwanted side effects. Conventionally panels of individual enzyme assays are used to assess the inhibitory effect of a compound for protein dioxygenases (McDonough et al., 2005; J. Am. Chem. Soc. 127(21): 7680-7681).

The *in vitro* investigation of dioxygenase activity is typically performed using enzyme assays. For example, the recombinant dioxygenases FIH and PHD2 were expressed in E. coli and tested in enzyme assays. Interestingly, the truncation of the PHD2 protein was found to be necessary in order to produce soluble highly active enzyme in E. coli (McDonough et al., 2005; J. Am. Chem. Soc. 127(21): 7680-7681). However, it can be advantageous to use endogenous full-length dioxygenases as isolated from mammalian cells for the identification of inhibitors.

Several reports described dioxygenase inhibitors (WO2009/049112; Nagel et al, 2010; Antioxid Redox Signal. 2010 Apr;12(4):481-501).

In view of the above, there is a need for providing effective tools and methods for the identification and selectivity profiling of dioxygenase interacting compounds as well as for the purification of dioxygenases.

In one aspect, the present invention relates to an immobilization compound according to formula (I) 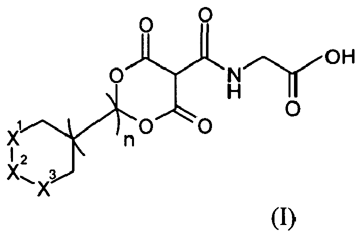 wherein each of X¹, X², and X³ is CH₂ or NH, with the proviso that the piperidinyl ring has only one NH-group as a ring member, and
wherein
n is 0 or 1;
or a salt thereof.

In a preferred embodiment, the present invention relates inter alia to an immobilization compound selected from the group consisting of formula (II) to formula (IV) 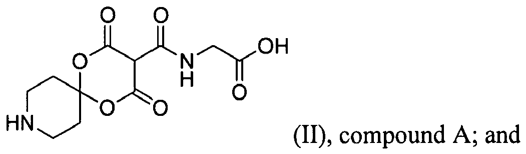 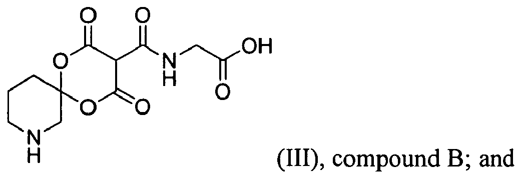 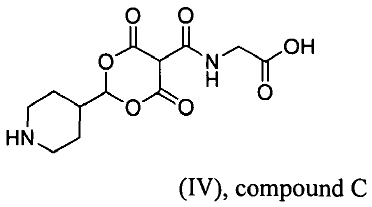 or a salt thereof.

In case the immobilization compounds according to formula (I) to formula (IV) contain one or more acidic or basic groups, the invention also comprises their corresponding salts. Thus, the immobilization compounds according to formula (I) to formula (IV) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds according to formula (I) to formula (IV) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the immobilization compounds according to formula (I) to formula (IV) simultaneously contain acidic and basic groups in the molecule, the invention also includes, in addition to the salt forms mentioned, inner salts or betaines (zwitterions). The respective salts according to formula (I) to formula (IV) can be obtained by customary methods which are known to the person skilled in the art like, for example by contacting these with an organic or inorganic acid or base in a solvent or dispersant, or by anion exchange or cation exchange with other salts.

The immobilization compounds of the present invention can be prepared by methods well known in the art.

Exemplary routes for the preparation of compounds of the present invention are described below. It is clear to a practitioner in the art to combine or adjust such routes especially in combination with the introduction of activating or protective chemical groups.

General routes for the preparation of exemplary compounds according to the present invention are outlined in Scheme 1.

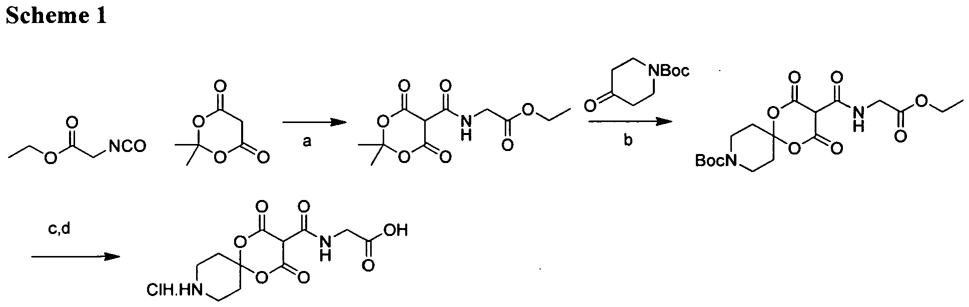

### a) DMF, Et₃N b) Toluene, Reflux c) NaOH 1N, EtOH d) HCl 4N in Dioxane.

The invention further relates to a method for the preparation of an immobilization product, wherein at least one immobilization compound according to the invention is immobilized on a solid support. Such immobilization products obtainable by the method of the invention are, e.g., useful in the methods of the invention for the identification of dioxygenase interacting compounds or in diagnostic methods for the diagnosis of inflammatory diseases, proliferative diseases and metabolic diseases.

According to the method of the invention, at least one immobilization compound of the invention is immobilized on a solid support. Throughout the invention, the term "solid support" relates to every undissolved support being able to immobilize a small molecule ligand on its surface.

According to the invention, the term "at least one immobilization compound" means either that at least one immobilization compound of the same type is immobilized on the solid support or that one or more different immobilization compounds (each of them either in singular or plural) may be immobilized on the solid support. Preferably, one or two different immobilization compounds are immobilized on the solid support.

The solid support may be selected from the group consisting of agarose, modified agarose, sepharose beads (e.g. NHS-activated sepharose), latex, cellulose, and ferro- or ferrimagnetic particles.

In case that the solid support is a material comprising various entities, e.g. in case that the solid support comprises several beads or particles, it is envisaged within the present invention that, if different immobilization compounds are immobilized, on each single entity, e.g. each bead or particle, one or more different immobilization compounds are immobilized. Therefore, in case that two immobilization compounds are used, it is envisaged within the present invention that on each single entity one or two different immobilization compounds are immobilized. If no measures are taken that on one entity only one different immobilization compound is immobilized, it is very likely that on each entity all different immobilization compounds will be present.

The immobilization compound or compounds of the invention may be coupled to the solid support either covalently or non-covalently. Non-covalent binding includes binding via biotin-affinity-ligand binding to steptavidin matrices.

Preferably, the immobilization compound or compounds are covalently coupled to the solid support.

Methods for immobilizing compounds on solid supports are known in the art and further exemplified in the Example section. Especially, the skilled person will understand that, if the immobilization compound is covalently coupled to said solid support, the residues forming part of said coupling will have to be modified accordingly.

In general, before the coupling, the matrixes can contain active groups such as NHS, Carbodiimide etc. to enable the coupling reaction with the immobilization compound. The immobilization compound can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to the immobilization product of the invention and non-covalent binding of biotin to streptavidin which is bound directly to the solid support).

The linkage to the solid support material may involve cleavable and non-cleavable linkers. The cleavage may be achieved by enzymatic cleavage or treatment with suitable chemical methods.

Therefore, according to a preferred embodiment of the invention, the immobilization product results from a covalent direct or linker mediated attachment of the at least one immobilization compound of the invention to the solid support. This linker may be a C₁₋₁₀ alkylene group, which is optionally interrupted or terminated by one or more atoms or functional groups selected from the group consisting of S, O, NH, C(O)O, OC(O), C(O), NHC(O), and C(O)NH and wherein the linker is optionally substituted with one or more substituents independently selected from the group consisting of halogen, OH, NH₂, C(O)H, C(O)NH₂, SO₃H, NO₂, and CN.

The term "C₁₋₁₀ alkylene" means an alkylene chain having 1 - 10 carbon atoms, e.g. methylene, ethylene, n-propylene and the like, wherein each hydrogen of a carbon atom may be replaced by a substituent as indicated herein. The term "C₁₋₆ alkylene" as used herein is defined accordingly.

The term "interrupted" means that the one or more atoms or functional groups are inserted between two carbon atoms of the alkylene chain or -when "terminated"- at the end of said chain.

The invention further relates to an immobilization product, obtainable by the method of the invention.

Furthermore, the present invention relates to an immobilization product, comprising the immobilization compound of the invention immobilized on a solid support, in particular wherein the solid support is selected from the group consisting of agarose, modified agarose, sepharose beads (e.g. NHS-activated sepharose), latex, cellulose, and ferro- or ferrimagnetic particles.

Methods and strategies for choosing appropriate solid supports and for coupling compounds to said solid supports are known in the art (Wong, Shan S., Chemistry of protein conjugation and cross-linking (1991), CRC Press, Inc. ISBN 0-8493-5886-8 Chapter 12: Conjugation of proteins to solid matrices, pages 295-318).

Therefore, an immobilization product which is obtainable by the method of the invention is or comprises an immobilization compound of the present invention immobilized on a solid support. This immobilization product will be referred to in the following as the immobilization product of the invention and is used in the methods of the present invention.

In a preferred embodiment, the immobilization compound or immobilization product of the invention may further be labeled.

By "labeled" is meant that the respective substance is either directly or indirectly labeled with a molecule which provides a detection signal, e.g. a radioisotope, fluorescent tag, chemiluminescent tag, a peptide or specific binding molecules. Specific binding molecules include pairs, such as biotin and streptavidin, digoxin and antidigoxin. The label can directly or indirectly provide a detectable signal. The tag can also be a peptide which can be used, for example, in an enzyme fragment complementation assay (e.g. betagalactosidase enzyme fragment complementation; Zaman et al., 2006; Assay Drug Dev. Technol. 4(4): 411-420). The labeled compounds would be useful not only in imaging techniques but also in assays, both in vitro and in vivo, for identifying dioxygenase interacting compounds by inhibition of binding of the labeled compound, for example in dioxygenase assays that contain such labeled compounds.

Radioisotopes are commonly used in biological applications for the detection of a variety of biomolecules and have proven to be useful in binding assays. Several examples of probes have been designed to incorporate ³H (also written as T for tritium) because it can replace hydrogen in a probe without altering its structure (Fenteany et al., 1995; Science 268: 726-731). An "isotopically" or "radio-labeled" compound is a compound of the invention where one or more atoms are replaced or substituted by an atom having an atomic mass or mass number different from the atomic mass or mass number typically found in nature (i.e., naturally occurring). Suitable radionuclides that may be incorporated in compounds of the present invention include, but are not limited to, ²H (also written D for Deuterium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I.

Guidance for the selection and methods for the attachment of fluorescent tags (e.g. fluorescein, rhodamine, dansyl, NBD (nitrobenz-2-oxa-1,3-diazole), BODIPY (dipyrromethene boron difluoride), and cyanine (Cy)-dyes) to small molecule ligands are generally known in the art (Vedvik et al., 2004; Assay Drug Dev. Technol. 2(2): 193-203; Zhang et al., 2005; Analytical Biochemistry 343(1): 76-83). The application of fluorescent probes (fluorophores) in assays for high throughput screening (HTS) of protein dioxygenases was described (Zaman et al., 2003; Comb. Chem. High Throughput Screen 6(4): 313-320). The change of the fluorescent properties after binding of the fluorescent probe to the target dioxygenase can be determined by means of fluorescent detection, including, but not limited to, measuring of fluorescence polarization (Kashem et al., 2007; J. Biomol. Screening 12(1):70-83), fluorescence resonance energy transfer (FRET; Zhang et al., 2005; Analytical Biochemistry 343(1):76-83), or measuring of fluorescence lifetime (Moger et al., 2006; J. Biomol. Screening 11(7): 765-772). In addition, the ALPHAScreen technology can be used where the excitation of a donor bead at 680 nm produces singlet oxygen which can diffuse to an acceptor bead undergoing a chemiluminescent reaction (Glickman et al., 2002; J. Biomol. Screen. 7(1): 3-10).

One possible use of the immobilization products of the invention is in the context of the identification of compounds interacting with dioxygenases. Therefore, the present invention also relates to such methods and uses.

In a first aspect of the methods of the invention, the invention therefore relates to a method for the identification of a dioxygenase interacting compound, comprising the steps of
a) providing a protein preparation containing at least one dioxygenase,
b) contacting the protein preparation with the immobilization product of the invention under conditions allowing the formation of one or more different complexes between one of the dioxygenases and the immobilization product,
c) incubating the one or more different complexes with a given compound, and
d) determining whether the compound is able to separate the dioxygenase from the immobilization product.

In a second aspect, the present invention relates into a method for the identification of a dioxygenase interacting compound, comprising the steps of
a) providing a protein preparation containing at least one dioxygenase,
b) contacting the protein preparation with the immobilization product of the invention and with a given compound under conditions allowing the formation of one or more different complexes between one of the dioxygenases and the immobilization product, and
c) detecting the complex or the complexes formed in step b).

In a third aspect, the present invention relates to a method for the identification of a dioxygenase interacting compound, comprising the steps of:
a) providing two aliquots of a protein preparation containing at least one dioxygenase,
b) contacting one aliquot with the immobilization product of the invention under conditions allowing the formation of one or more different complexes between one of the dioxygenases and the immobilization product,
c) contacting the other aliquot with the immobilization product of the invention and with a given compound under conditions allowing the formation of one or more different complexes between one of the dioxygenases and the immobilization product, and
d) determining the amount of the complex or the complexes formed in steps b) and c).

In a fourth aspect, the invention relates to a method for the identification of a dioxygenase interacting compound, comprising the steps of:
a) providing two aliquots of a cell preparation comprising each at least one cell containing at least one dioxygenase,
b) incubating one aliquot with a given compound,
c) harvesting the cells of each aliquot,
d) lysing the cells in order to obtain protein preparations,
e) contacting the protein preparations with the immobilization product of the invention under conditions allowing the formation of one or more different complexes between one of the dioxygenases and the immobilization product, and
f) determining the amount of the complex or the complexes formed in each aliquot in step e).

In the context of the present invention, it has been found that the immobilization products of the present invention are suitable for the identification of compounds interacting with dioxygenases.

The immobilization products of the present invention bind several dioxygenases. For example, the following dioxygenases were identified in the biological examples (Tables 3, 6, 7 and 8): ALKBH2, ALKBH3, ALKBH5, ALKBH7, C17ORF101, EGLN1, EGLN2, FTO, LEPRE1, LEPREL2, OGFOD1, OGFOD2, P4HA1, P4HA2, PLOD1, PLOD2, PLOD3, and TMLHE.

According to the present invention, the expression "dioxygenase" denotes an enzyme that metabolizes molecular oxygen (dioxygen or O₂) and incorporates both atoms of oxygen into its products.

Preferably, the enzyme belongs to the family of the 2-oxoglutarate (2OG)- and Fe(II)-dependent dioxygenases (2OG-Fe(II) dioxygenases). The catalytic core of the proteins consists of a double-stranded β-helix (DSBH) fold containing a HX[DE] dyad (where X is any amino acid) and a conserved carboxyterminal histidine which together chelate a single iron atom. The 2OG-Fe(II) dioxygenase family comprises approximately 60 members in humans (Aravind and Koonin, 2001; Genome Biol. 2(3): RESEARCH0007-research0007.8).

Consequently, in the context of the present invention, the term "at least one dioxygenase" means that at least one type of dioxygenase (e.g. ALKBH2, ALKBH3, ALKBH5, ALKBH7, C17ORF101, EGLN1, EGLN2, FTO, LEPRE1, LEPREL2, OGFOD1, OGFOD2, P4HA1, P4HA2, PLOD1, PLOD2, PLOD3, or TMLHE) is present in the respective sample, e.g. the cell or the protein preparation.

Furthermore, the skilled person will appreciate that the respective sample will contain one or more dioxygenases of said type. Therefore, the person skilled in the art will understand that in the context of the present invention, with respect to a dioxygenase, the singular and the plural may be used interchangeably.

Consequently, in the methods of the present invention, these immobilization products can be used to identify compounds binding to dioxygenases.

According to the present invention, the expression "dioxygenase" relates to both human and other proteins of this family. The expression especially includes functionally active derivatives thereof, or functionally active fragments thereof, or homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

In the context of the present invention, the term "functionally active derivative or fragment thereof" generally means any kind of protein revealing enzymatic activity of a dioxygenase that comprises the amino acid sequence of a naturally occurring protein with dioxygenase activity in any partially, substituted or modified form. That is, a functionally active derivative or fragment according to the present invention might be comprised of protein domain(s) originated from a naturally occurring protein with dioxygenase activity, characterized in that it reveals the same enzymatic activity as the wild type protein it derived from. Such a functionally active fragment or derivative might be constituted of any N-terminal, C-terminal or central protein domain of interest, or of any combination thereof.

A functionally active derivative or fragment of the present invention may further be characterized by structural features. Accordingly, in a preferred embodiment of the invention, a functionally active fragment or derivative reveals at least 70 %, preferably at least 80 %, more preferably at least 90 %, even more preferably at least 95 %, and most preferably 99 % sequence identity to a naturally occurring protein with enzymatic activity of a dioxygenase. The percentage of sequence identity can be determined by, e.g., sequence alignment. Methods of how to align sequences for comparison are well known by the person skilled in the art.

Moreover, according to the present invention, the expression "dioxygenase" includes mutant forms of said dioxygenases.

In some aspects of the invention, first a protein preparation containing said dioxygenase is provided. The methods of the present invention can be performed with any protein preparation as a starting material, as long as the respective dioxygenase is solubilized in the preparation. Examples include a liquid mixture of several proteins, a cell lysate, a partial cell lysate which contains not all proteins present in the original cell or a combination of several cell lysates. The term "protein preparation" also includes dissolved purified protein.

In another aspect of the invention, aliquots of a cell preparation are provided as the starting material. In the context of the present invention, the term "cell preparation" refers to any preparation containing at least one cell with the desired properties. Suitable cell preparations are described below.

The presence of the dioxygenases in a protein preparation of interest can be detected on Western blots probed with antibodies that are specifically directed against said dioxygenase. Alternatively, also mass spectrometry (MS) could be used to detect the dioxygenases (see below).

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art (Chapter 4.2 Purification of Organelles from Mammalian Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein preparations can be prepared by fractionation of cell extracts thereby enriching specific types of proteins such as nuclear proteins (Dignam et al., 1983; Nucleic Acids Res. 11(5): 1475-89).

Furthermore, the protein preparation may be a preparation containing the dioxygenase or the dioxygenases which has/have been recombinantely produced. Methods for the production of recombinant proteins in prokaryotic and eukaryotic cells are widely established (Chapter 5 Production of Recombinant Proteins in "Current Protocols in Protein Science", Editors: John. E. Coligan, Ben M. Dunn, Hidde L. Ploegh, David W. Speicher, Paul T. Wingfield; Wiley, 1995, ISBN: 0-471-14098-8).

In a preferred embodiment of the methods of the invention, the provision of a protein preparation includes the steps of harvesting at least one cell containing the dioxygenase or the dioxygenases and lysing the cell.

Suitable cells for this purpose as well as for the cell preparations used as the starting material in one aspect of the present invention are e.g. those cells or tissues where the dioxygenases are expressed. In any given cell or tissue only a subset of the dioxygenases may be expressed. Therefore it may be necessary to generate multiple protein preparations from a variety of cell types and tissues to cover the dioxygenase family of proteins, especially for selectivity profiling of dioxygenase inhibitors. As established cell lines may not reflect the physiological expression pattern of dioxygenases, primary animal or human cells may be used, for example cells isolated from blood samples.

Therefore, in a preferred embodiment, cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection).

As an alternative to primary human cells cultured cell lines (e.g. MOLT-4 cells, Jurkat, Ramos, HL-60 or HeLa cells) can be used.

In a preferred embodiment, the cell is part of a cell culture system and methods for the harvest of a cell out of a cell culture system are known in the art (literature supra).

The choice of the cell will mainly depend on the expression of the dioxygenases, since it has to be ensured that the protein is principally present in the cell of choice. In order to determine whether a given cell is a suitable starting system for the methods of the invention, methods like Westernblot, PCR-based nucleic acids detection methods, Northernblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyzed then cells or tissues may be selected in which the desired therapeutic effect occurs (e.g. T-cells). By contrast, for the elucidation of protein targets mediating unwanted side effects the cell or tissue may be analysed in which the side effect is observed (e.g. cardiomyocytes).

Furthermore, it is envisaged within the present invention that the cell containing the dioxygenases or the dioxygenase may be obtained from an organism, e.g. by biopsy. Corresponding methods are known in the art. For example, a biopsy is a diagnostic procedure used to obtain a small amount of tissue, which can then be examined microscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

According to the methods of the invention, the protein preparation containing one or more dioxygenase(s) is contacted with the immobilization product under conditions allowing the formation of a complex between the said dioxygenase and the immobilization product of the invention.

In the present invention, the term "a complex between a dioxygenase and the immobilization product" denotes a complex where the immobilization product interacts with a dioxygenase , e.g. by covalent or, most preferred, by non-covalent binding.

In the context of the present invention, compounds are identified which interfere with the formation of a complex between the immobilization product and a dioxygenase present in a cell or protein preparation. In case that only one dioxygenase is to be detected or present, the formation of one complex is observed and tested. In case that several dioxygenases are to be detected or present, the formation of several, different complexes is observed and tested.

The skilled person will know which conditions can be applied in order to enable the formation of said complex.

In the context of the present invention, the term "under conditions allowing the formation of the complex" includes all conditions under which such formation, preferably such binding is possible. This includes the possibility of having the solid support on an immobilized phase and pouring the lysate onto it. In another preferred embodiment, it is also included that the solid support is in a particulate form and mixed with the cell lysate. Such conditions are known to the person skilled in the art.

In the context of non-covalent binding, the binding between the immobilization product and the dioxygenase is, e.g., via salt bridges, hydrogen bonds, hydrophobic interactions or a combination thereof.

In a preferred embodiment, the steps of the formation of said complex are performed under essentially physiological conditions. The physical state of proteins within cells is described in Petty, 1998 (Howard R. Petty, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001Print Publication Date: October, 1998).

The contacting under essentially physiological conditions has the advantage that the interactions between the ligand, the cell preparation (i. e. the dioxygenase to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCl, pH 6.5-8.5, 20-37°C, and 0.001-10 mM divalent cation (e.g. Mg++, Ca++,); more preferably about 150 m NaCl or KCl, pH 7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2 %, typically 0.05-0.2 % (volume/volume). For general guidance, the following buffered aequous conditions may be applicable: 10-250 mM NaCl, 5-50 mM Tris HCl, pH 5-8, with optional addition of divalent cation(s) and/or metal chelators and/or non-ionic detergents.

Preferably, "essentially physiological conditions" mean a pH value from 6.5 to 7.5, preferably from 7.0 to 7.5, and / or a buffer concentration from 10 to 50 mM, preferably from 25 to 50 mM, and / or a concentration of monovalent salts (e.g. Na or K) from 120 to 170 mM, preferably of 150 mM. Divalent salts (e.g. Mg or Ca) may further be present at a concentration from 1 to 5 mM, preferably of 1 to 2 mM, wherein more preferably the buffer is selected from the group consisting of Tris-HCl or HEPES.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the identification methods of the invention, the read-out system is either the detection or determination of a dioxygenase (first aspect of the invention), the detection of the complex between a dioxygenase and the immobilization product (second aspect of the invention), or the determination of the amount of the complex between a dioxygenase and the immobilization product (second, third and fourth aspect of the invention).

In the method according to the first aspect of the invention, the detection or determination of the amount of separated dioxygenase is preferably indicative for the fact that the compound is able to separate the dioxygenase from the immobilization product. This capacity indicates that the respective compound interacts, preferably binds to the dioxygenase, which is indicative for its therapeutic potential.

In one embodiment of the method according to the second aspect of the invention, the complex formed during the method of the invention is detected. The fact that such complex is formed preferably indicates that the compound does not completely inhibit the formation of the complex. On the other hand, if no complex is formed, the compound is presumably a strong interactor with the dioxygenase, which is indicative for its therapeutic potential.

According to the methods of the second, third and fourth aspect of the invention the amount of the complex formed during the method is determined. In general, the less complex in the presence of the respective compound is formed, the stronger the respective compound interacts with the dioxygenase, which is indicative for its therapeutic potential.

The detection of the complex formed according to the second aspect of the invention can be performed by using labeled antibodies directed against the dioxygenase and a suitable readout system.

According to a preferred embodiment of the second aspect of the invention, the complex between one dioxygenase and the immobilization product is detected by determining its amount.

In the course of the second, third and fourth aspect of the invention, it is preferred that the dioxygenase are separated from the immobilization product in order to determine the amount of said complex.

According to invention, separating means every action which destroys the interactions between the immobilization compound and the dioxygenase. This includes in a preferred embodiment the elution of the dioxygenase from the immobilization compound.

The elution can be achieved by using non-specific reagents as described in detail below (ionic strength, pH value, detergents). In addition, it can be tested whether a compound of interest can specifically elute the dioxygenase from the immobilization compound. Such dioxygenase interacting compounds are described further in the following sections.

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized compound. The application of an elution buffer can dissociate binding partners by extremes of pH value (high or low pH; e.g. lowering pH by using 0.1 M citrate, pH2-3), change of ionic strength (e.g. high salt concentration using NaI, KI, MgCl₂, or KCl), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinty chromatography).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

In step d) of the method according to the first aspect of the invention, it is determined if the dioxygenase has been separated from the immobilization product of the invention. This may include the detection of the dioxygenase or the determination of the amount of the dioxygenase.

Consequently, at least in preferred embodiments of all identification methods of the invention, methods for the detection of a separated dioxygenase or for the determination of their amount are used. Such methods are known in the art and include physico-chemical methods such as protein sequencing (e.g. Edmann degradation), analysis by mass spectrometry methods or immunodetection methods employing antibodies directed against the dioxygenase.

Throughout the invention, if an antibody is used in order to detect a dioxygenase or in order to determine its amount (e.g. via ELISA), the skilled person will understand that, if a specific dioxygenase is to be detected or if the amount of a dioxygenase is to be determined, a specific antibody may be used (Appellhoff et al., 2004. J. Biol. Chem. 279(37):38458-65). As indicated above, such antibodies are known in the art. Furthermore, the skilled person is aware of methods for producing the same.

Preferably, a dioxygenase is detected or the amount of a dioxygenase is determined by mass spectrometry or immunodetection methods.

The identification of proteins with mass spectrometric analysis (mass spectrometry, MS) is known in the art (Shevchenko et al., 1996, Analytical Chemistry 68: 850-858; Mann et al., 2001; Annual Review of Biochemistry 70, 437-473) and is further illustrated in the example section.

Preferably, the mass spectrometry analysis is performed in a quantitative manner, for example by using iTRAQ technology (isobaric tags for relative and absolute quatification) or cICAT (cleavable isotope-coded affinity tags) (Wu et al., 2006; J. Proteome Res. 5, 651-658).

According to a further preferred embodiment of the present invention, the characterization by mass spectrometry is performed by the identification of proteotypic peptides of the dioxygenase. The idea is that the dioxygenase is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given dioxygenase, it is possible to compare the proteotypic peptides obtained for a given sample with the proteotypic peptides already known for dioxygenases and thereby identifying the dioxygenase being present in the sample.

As an alternative to mass spectrometry analysis, the eluted dioxygenase (including coeluted binding partners such as regulatory subunits), can be detected or its amount can be determined by using a specific antibody directed against the dioxygenase.

Furthermore, in another preferred embodiment, once the identity of the coeluted binding partner (e.g. regulatory subunit) has been established by mass spectrometry analysis, each binding partner can be detected with specific antibodies directed against this protein.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a dioxygenase interacting protein of interest, for example a component of a dioxygenase protein complex

(Appellhoff et al., 2004; J. Biol. Chem. 279(37): 38458-65), but also to analyse posttranslational modification patterns such as phosphorylation, hydroxylation or ubiquitin modification.

Furthermore, the identification methods of the invention involve the use of compounds which are tested for their ability to be a dioxygenase interacting compound.

Principally, according to the present invention, such a compound can be every molecule which is able to interact with the dioxygenase, eg. by inhibiting its binding to the immobilization product of the invention. Preferably, the compound has an effect on the dioxygenase, e.g. a stimulatory or inhibitory effect.

Preferably, said compound is selected from the group consisting of synthetic or naturally occurring chemical compounds or organic synthetic drugs, more preferably small molecule organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H., Natural product guided compound library development. Curr. Med. Chem. 2002; 9(23): 2129-2145, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. The current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the second and third aspect of the invention, the dioxygenase containing protein preparation is first incubated with the compound and then with the immobilization product. However, the simultaneous incubation of the compound and the immobilization product of the invention (coincubation) with the dioxygenase containing protein preparation is equally preferred (competitive binding assay).

In case that the incubation with the compound is first, the dioxygenase is preferably first incubated with the compound for 10 to 60 minutes, more preferred for 30 to 45 minutes at a temperature of 4°C to 37°C, more preferred at a temperature of 4°C to 25°C, most preferred at 4°C. Preferably compounds are used at concentrations ranging from 1 nM to 1 mM, preferably ranging from 1 nM to 100 µM, more preferably ranging from 1 nM to 10 µM. The second step, contacting with the immobilized ligand, is preferably performed for 10 to 60 minutes at 4°C.

In case of simultaneous incubation, the dioxygenase is preferably simultaneously incubated with the compound and the immobilization product of the invention for 30 to 120 minutes, more preferred for 60 to 120 minutes at a temperature of 4°C to 37°C, more preferred at a temperature of 4°C to 25°C, most preferred at 4°C. Preferably compounds are used at concentrations ranging from 1 nM to 1 mM, preferably ranging from 1 nM to 100 µM, more preferably ranging from 1 nM to 10µM.

Furthermore, steps a) to c) of the second aspect of the invention may be performed with several protein preparations in order to test different compounds. This embodiment is especially interesting in the context of medium or high throughput screenings.

In a preferred embodiment of the method of the invention according to the third or fourth aspect, the amount of the complex formed in step c) is compared to the amount formed in step b).

In a preferred embodiment of the method of the invention according to the third or fourth aspect, a reduced amount of the complex formed in step c) in comparison to step b) indicates that a dioxygenase is a target of the compound. This results from the fact that in step c) of this method of the invention, the compound competes with the immobilized compound for the binding of the dioxygenase. If less dioxygenase is present in the aliquot incubated with the compound, this means preferably that the compound has competed with the inhibitor for the interaction with the enzyme and is, therefore, a direct target of the protein and vice versa.

Preferably, the identification methods of the invention are performed as a medium or high throughput screening.

The interaction compound identified according to the present invention may be further characterized by determining whether it has an effect on the dioxygenase, for example on its dioxygenase activity (McDonough et al., 2005; J. Am. Chem. Soc. 127(21): 7680-7681).

The compounds identified according to the present invention may further be optimized in terms of potency and selectivity. An example for lead optimization of dioxygenase inhibitors was reported (McDonough et al., 2005. J. Am. Chem. Soc. 127(21):7680-7681).

The invention further relates to a method for the preparation of a pharmaceutical composition comprising the steps of
a) identifying a dioxygenase interacting compound as described above, and
b) formulating the interacting compound to a pharmaceutical composition.

Methods for the formulation of identified compounds are known in the art. Furthermore, it is known in the art how to administer such pharmaceutical compositions.

The obtained pharmaceutical composition can be used for the prevention or treatment of diseases where the respective dioxygenase plays a role, e.g. for the prevention or treatment of cancer. For example, dioxygenase inhibitors may be useful for the treatment of inflammatory diseases, cancer or metabolic diseases.

The invention further relates to a method for the purification of a dioxygenase, comprising the steps of
a) providing a protein preparation containing said dioxygenase,
b) contacting the protein preparation with the immobilization product of the invention under conditions allowing the formation of a complex between the dioxygenase and the immobilization product, and
c) separating the dioxygenase from the immobilization product.

As mentioned above, it has been surprisingly found that the compound of the invention and therefore also the immobilization product of the invention is a ligand which recognizes the dioxygenases mentioned above. This enables efficient purification methods for said dioxygenases.

With respect to the dioxygenases, the protein preparation containing the dioxygenases, the conditions for contacting with the immobilization product of the invention, the immobilization product of the invention, the complex between the dioxygenases and the immobilization product of the invention, the separation of the dioxygenases from the immobilization product of the invention, and the detection of the dioxygenases or the determination of its amount, the embodiments as defined above for the identification methods of the invention also apply to the purification method of the invention.

In a preferred embodiment, the purification method of the invention further comprises after step c) the identification of proteins being capable of binding to said dioxygenases. This is especially interesting when the formation of the complex is performed under essentially physiological conditions, because it is then possible to preserve the natural condition of the enzyme which includes the existence of binding partners, enzyme subunits or post-translational modifications, which can then be identified with the help of mass spectrometry.

Consequently, in a preferred embodiment, the purification method of the invention further comprises after step c) the determination whether the dioxygenase is further posttranslationally modified, e.g. by ubiquitin modification.

The binding proteins or the posttranslational modifications can be determined as explained above for the detection of dioxygenases or the determination of the amount of dioxygenases. Preferably, said methods include mass spectrometry of immunodetection methods as described above.

The invention further relates to a method for determining the presence of one or more dioxygenases in a sample, comprising the steps of:
a) providing a protein preparation expected to contain said one or more dioxygenases,
b) contacting the protein preparation with the immobilization product of the invention under conditions allowing the formation of a complex between one of the dioxygenases and the immobilization product, and
c) detecting whether one or more dioxygenases have formed a complex with the immobilization product.

In a preferred embodiment of the invention, said detecting in step c) is performed by separating said one or more dioxygenases from the immobilization product and further identification of said one or more dioxygenases.

Said identification may be performed by mass spectrometry or immunodetection methods as described above.

According to an especially preferred embodiment of this method of the invention, the dioxygenase contains at least one mutation.

With respect to said one or more dioxygenases, the protein preparation containing said dioxygenases, the conditions for contacting with the immobilization product of the invention, the immobilization product of the invention, the complex between said dioxygenase and the immobilization product of the invention, the separation of dioxygenases from the immobilization product of the invention, and the detection of dioxygenases or the determination of its amount, the embodiments as defined above for the identification methods of the invention also apply to the purification method of the invention.

The invention further relates to the use of the immobilization compound or the immobilization product of the invention for the identification of a dioxygenase interacting compound and for the purification of a dioxygenase. The embodiments as defined above also apply to the uses of the invention.

The invention further relates to a kit comprising the compound or the immobilization product of the invention. Such a kit is especially useful for performing the methods of the invention. Further components of the kit may be antibodies for the detection of dioxygenase proteins. Such antibodies and their use are known in the art and they are commercially available (Appellhoff et al., 2004; J. Biol. Chem. 279(37): 38458-65). Furthermore, the kit may contain further auxiliary components like buffers, means for the detection of antibodies, and positive controls. Such components are known in the art.

The invention is further illustrated by the following figures and examples, which are not considered as being limiting for the scope of protection conferred by the claims of the present application. In case where in the following examples the term "affinity matrix" is used, this term refers to an immobilization product as defined in the present application.

### Brief description of the figures

**Figure 1**: Amino acid sequence of human PLOD 1 (IPI00027192.6). Peptides identified by mass spectrometry are underlined.
**Figure 2**: Amino acid sequence of human PLOD3 (IPI00030255.1). Peptides identified by mass spectrometry are underlined.
**Figure 3****:** Amino acid sequence of human LEPRE1 (IPI00045839.3). Peptides identified by mass spectrometry are underlined.
**Figure 4****:** Amino acid sequence of human ALKBH2 (IPI00055405.3). Peptides identified by mass spectrometry are underlined.
**Figure 5****:** Amino acid sequence of human ALKBH3 (IPI00076597.3). Peptides identified by mass spectrometry are underlined.
**Figure 6****:** Amino acid sequence of human OGFOD1 (IPI00170429.3). Peptides identified by mass spectrometry are underlined.
**Figure 7****:** Amino acid sequence of human LEPREL2 (IPI00217056.2). Peptides identified by mass spectrometry are underlined.
**Figure 8**: Amino acid sequence of human C17ORF101 (ISOFORM 2 OF PKHD DOMAIN-CONTAINING TRANSMEMBRANE PROTEIN FLJ22222) (IPI00217869.4). Peptides identified by mass spectrometry are underlined.
**Figure 9****:** Amino acid sequence of human P4HA1 (IPI00218682.1). Peptides identified by mass spectrometry are underlined.
**Figure 10****:** Amino acid sequence of human FTO (IPI00641635.1). Peptides identified by mass spectrometry are underlined.
**Figure 11****:** Amino acid sequence of human ALKBH5 (IPI00759562.1). Peptides identified by mass spectrometry are underlined.
**Figure 12****:** Amino acid sequence of human EGLN1 (IPI00004928.1). Peptides identified by mass spectrometry are underlined.

### Examples

### Example 1: Preparation of the affinity matrix

This example describes the synthesis of compounds and methods for their immobilization on a solid support yielding the affinity matrix used in the following examples for the capturing of proteins from cell lysates.

### Synthesis of ethyl 2-(2,2-dimethyl-4,6-dioxo-1,3-dioxane-5-carboxamido)acetate

Triethylamine (12mmol, 1.7ml) was added to a solution isopropylidene malonate (6mmol, 0.862g) in DMF (6ml). The reaction was stirred at room temperature for 5mn before adding the ethyl 2-isocyanatoacetate (6mmol, 0.685ml). The reaction was stirred at room temperature a further 1 hour, then poured on an ice cold 0.1M HCl (30ml). The resulting precipitate was collected by filtration, washed with ice cold water, and dried in a vacuum oven (40°C) to yield the desired product as a white solid (0.94g, 49%). uPLC (method A) rt=1.07mn, no mass.NMR DMSO= 1.21 (t, 3H), 1.67 (s, 6H), 4.16 (q, 2H), 4.2 (d, 2H), 9.6 (s, 1H).

### Synthesis of tert-butyl 3-((2-ethoxy-2-oxoethyl)carbamoyl)-2,4-dioxo-1,5-dioxa-9-azaspiro[5.5]undecane-9-carboxylate

Ethyl 2-(2,2-dimethyl-4,6-dioxo-1,3-dioxane-5-carboxamido)acetate (1mmol, 0.273g) and tert-butyl 4-oxopiperidine-1-carboxylate (2mmol, 0.400g) were dissolved in toluene (10ml). The reaction was stired at reflux for 2H. The solvent was removed and the resulting crude material purified by flash chromatography (Dichloromethane/ (0 to 7%) Methanol) to yield the desired product (0.418g, 100%).LCMS (method B) rt=3.23mn, M+Na=437.

### Synthesis of 2-(9-(tert-butoxycarbonyl)-2,4-dioxo-1,5-dioxa-9-azaspiro[5.5]undecane-3-carboxamido)acetic acid

tert-butyl 3-((2-ethoxy-2-oxoethyl)carbamoyl)-2,4-dioxo-1,5-dioxa-9-azaspiro[5.5]undecane-9-carboxylate (4.8mmol, 2g) was dissolved in Ethanol (70ml). NaOH 1N (12ml) was added and the reaction stirred at room temperature overnight. The mixture was filtered, diluted with water (150ml). 12ml of acetic acid were added and the mixture was extracted with Ethyl acetate. The organic layers were dried with MgSO4, filtered, the solvent was removed and the residue azeotroped 3 times with toluene. The oil was triturated with ether, and the solid collected (0.112g, 6%). The filtrate was concentrated and purified by preparative HPLC to yield another crop of desired product (0.270g, 15%). LCMS (method C) rt=9.17mn, M+Na=409. NMR DMSO 1.48 (s, 9H), 2.0 (m, 4H), 3.55 (m, 4H), 4.2 (s, 2H)

### Synthesis of 2-(2,4-dioxo-1,5-dioxa-9-azaspiro[5.5]undecane-3-carboxamido)acetic acid hydrochloride (immobilization compound A according to formula (II))

2-(9-(tert-butoxycarbonyl)-2,4-dioxo-1,5-dioxa-9-azaspiro[5.5]undecane-3-carboxamido)acetic acid (0.034mmol, 0.013g) was dissolved in dichloromethane. HCl 4N in dioxane (0.4ml) was added and the reaction stirred at room temperature for 2 hours. The solvent was then removed to yield the desired product as an off whiter solid (0.011 g, 100%). uPLC (method A) rt=0.4mn, M+H=287, M-H=285. LCMS (Method D) Rt=7.02mn. M+H=287, M-H=285

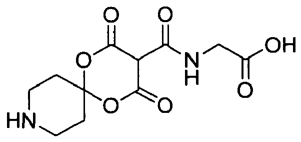

### Synthesis of 2-(2,4-dioxo-1,5-dioxa-8-azaspiro[5.5]undecane-3-carboxamido)acetic acid hydrochloride (immobilization compound B according to formula (III))

The compound was made in a manner similar to the one previously described for 2-(2,4-dioxo-1,5-dioxa-9-azaspiro[5.5]undecane-3-carboxamido)acetic acid hydrochloride, replacing tert-butyl 4-oxopiperidine-1-carboxylate with tert-butyl 3-oxopiperidine-1-carboxylate. uPLC (method A) RT=0.3mn M+H=287

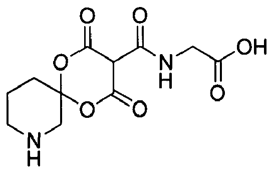

### Synthesis of 2-(4,6-dioxo-2-(piperidin-4-yl)-1,3-dioxane-5-carboxamido)acetic acid hydrochloride (immobilization compound C according to formula (IV))

The compound was made in a manner similar to the one previously described for 2-(2,4-dioxo-1,5-dioxa-9-azaspiro[5.5]undecane-3-carboxamido)acetic acid hydrochloride, replacing tert-butyl 4-oxopiperidine-1-carboxylate with ter-butyl 4-formylpiperidine-1-carboxylate. LCMS (method D) rt 6.95 mn M+H=301

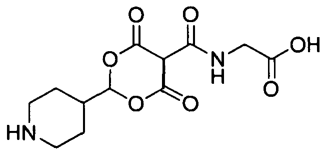

### Analytical methods

### uPLC

### 1. Columns

Short Column: Waters Acquity UPLC BEH C18, 2.1 x 30 mm, 1.7 microns
Long Column: Waters Acquity UPLC BEH C18, 2.1 x 50mm, 1.7 microns

### 2. Solvents

■ A1= Water with 0.1% formic acid
■ B1= Acetonitrile with 0.1% formic acid

### 3. Flow rate: 0.5 ml/min

### 4. Temperature: 40°C

### 5. Gradient conditions

### ■ Method A

| Time (min) | %A1 | %B1 |
|---|---|---|
| 0.00 | 95.0 | 5.0 |
| 0.20 | 95.0 | 5.0 |
| 1.00 | 5.0 | 95.0 |
| 1.50 | 5.0 | 95.0 |
| 1.70 | 95.0 | 5.0 |
| 2.70 | 95.0 | 5.0 |

### LCMS

### 1. Columns

Short column: Phenomenex Gemini-NX C 18, 3.0 x 30 mm, 3 microns
Long column: Phenomenex Gemini-NX C 18, 4.6 x 150 mm, 5 microns

### 2. Solvents

■ A= Water with 0.1 % formic acid
■ B= Acetonitrile with 0.1 % formic acid

### 3. Flow rate: 1.2 ml/min

### 4. Temperature: 40°C

### 5. Gradient conditions

### ■ Method B: Short Column Analytical, Low pH, Positive/Negative ion

| Time (min) | %A | %B |
|---|---|---|
| 0.00 | 95.0 | 5.0 |
| 3.00 | 5.0 | 95.0 |
| 4.50 | 5.0 | 95.0 |
| 4.60 | 95.0 | 5.0 |
| 6.00 | 95.0 | 5.0 |

### ■ Method C: Long Column Analytical, Low pH, Positive/Negative ion

| Time (min) | %A | %B |
|---|---|---|
| 0.00 | 95.0 | 5.0 |
| 11.00 | 5.0 | 95.0 |
| 13.00 | 5.0 | 95.0 |
| 13.01 | 95.0 | 5.0 |
| 16.00 | 95.0 | 5.0 |

### LCMS method D

### 1. Column:

Phenomenex Luna Hilic 150*4.6mm 5µm

### 2. Solvents:

A= 5mM Ammonium Acetate pH 5.8
B= 90%: 10%, Acetonitrile: 50mM Ammonium Acetate pH5.8

### 3. Flow Rate: 2.0ml/min

### 4. Temperature: room temperature

### 5. Gradient conditions:

| Time (min) | %A | %B |
|---|---|---|
| 0.00 | 0.0 | 100.0 |
| 2.50 | 0.0 | 100.0 |
| 10.00 | 50.0 | 50.0 |
| 12.50 | 50.0 | 50.0 |
| 13.00 | 0.0 | 100.0 |
| 20.00 | 0.0 | 100.0 |

**Table 1: Abbreviations**

| | |
|---|---|
| DMF | N,N-Dimethylformamide |
| ml | millilitres |
| rt | Retention time |
| g | gram |
| mmol | millimoles |
| HCl | Hydrochloric acid |
| NaOH | Sodium Hydroxyde |
| MgSO₄ | Magnesium sulfate |
| DMSO | Dimethylsulfoxide |

### Immobilization of compounds on beads (affinity matrix)

NHS-activated Sepharose 4 Fast Flow (Amersham Biosciences, 17-0906-01) was equilibrated with anhydrous DMSO (Dimethylsulfoxide, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in the following examples. Control beads (no compound immobilized) were generated by blocking the NHS-groups by incubation with aminoethanol as described above.

### Example 2: Bead assay using immobilized compound A and HL-60 nuclear extracts

This example demonstrates the use of immobilized compound A for the capturing and identification of dioxygenases from nuclear protein extracts in a competition binding assay. To the first aliquot of cell lysate 200 µM of the free compound A was added and allowed to bind to proteins in the lysate. Then the affinity matrix with the immobilized compound A was added to capture proteins that were not interacting with the previously added free compound. Beads were separated from the lysate and bead bound proteins were eluted in SDS sample buffer and subsequently separated by SDS-Polyacrylamide gel electrophoresis. Suitable gel bands were cut out and subjected to in-gel proteolytic digestion with trypsin. The second lysate aliquot was processed identically, however no free compound was added (DMSO solvent control). Peptides originating from samples 1 and 2 were labeled with iTRAQ reagents (iTRAQ 114 and iTRAQ 116) and the combined samples were analyzed with a nano-flow liquid chromatography system coupled online to a tandem mass spectrometer (LC-MS/MS) experiment followed by iTRAQ reporter ion quantification in the MS/MS spectra (Ross et al., 2004. Mol. Cell. Proteomics 3(12):1154-1169). Further experimental protocols can be found in WO2006/134056 and previous publications (Bantscheff et al., 2007. Nature Biotechnology 25, 1035-1044; Bantscheff et al., 2011. Nature Biotechnology 29, 255-265).

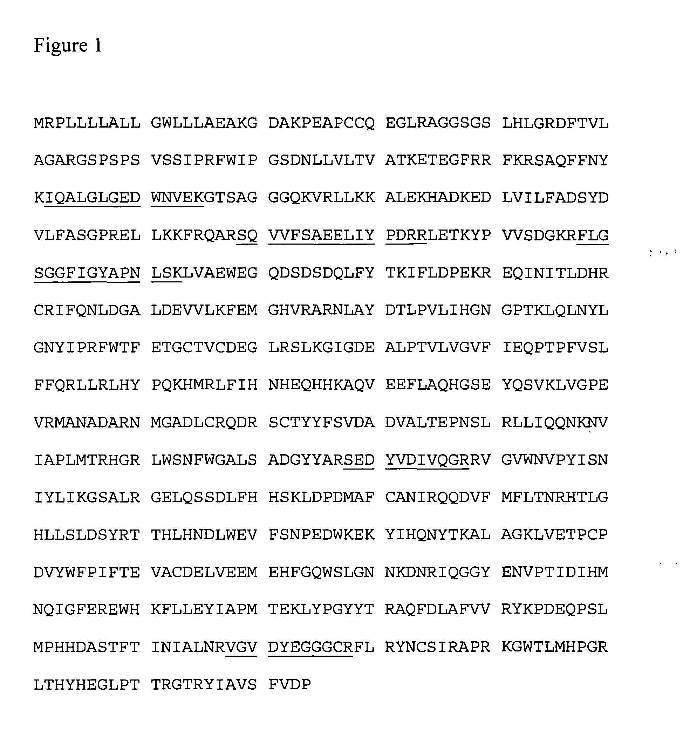

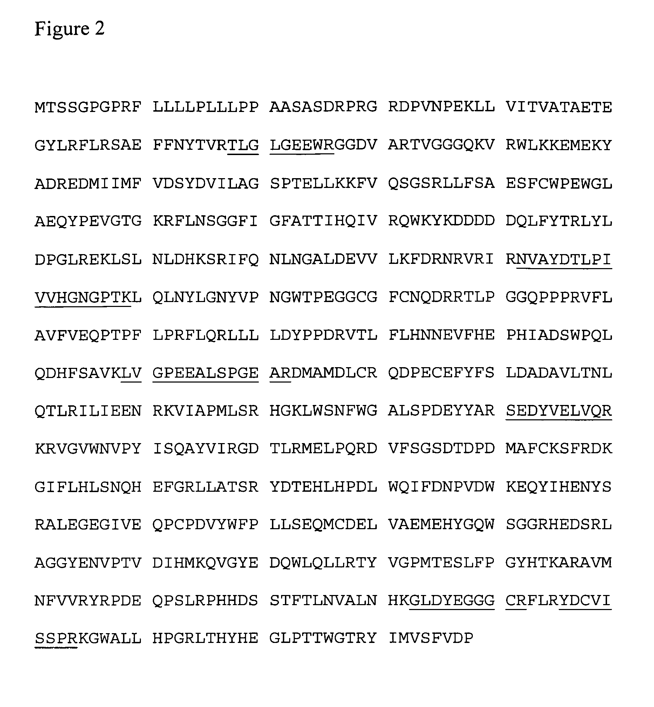

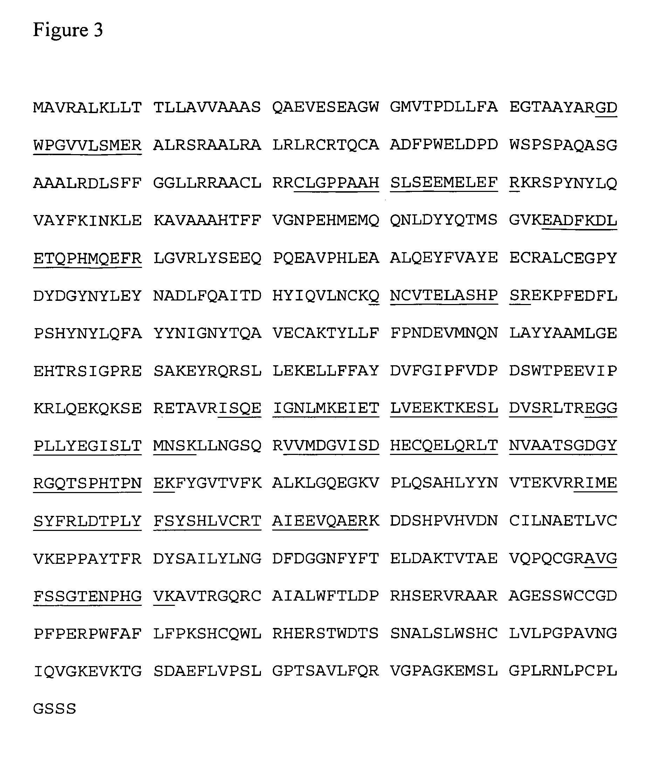

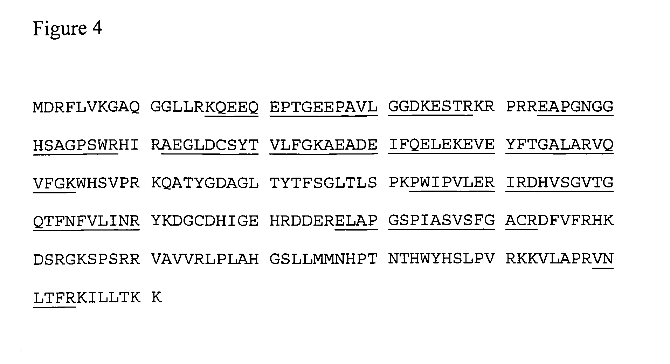

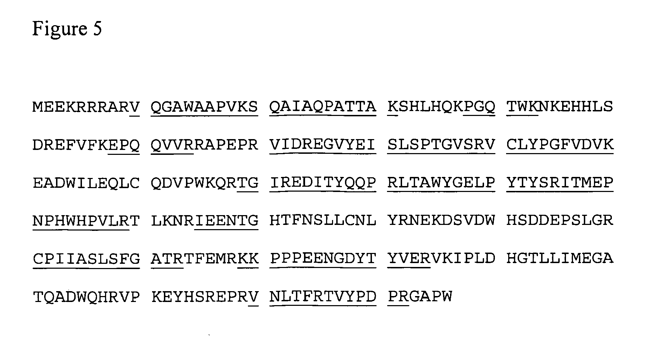

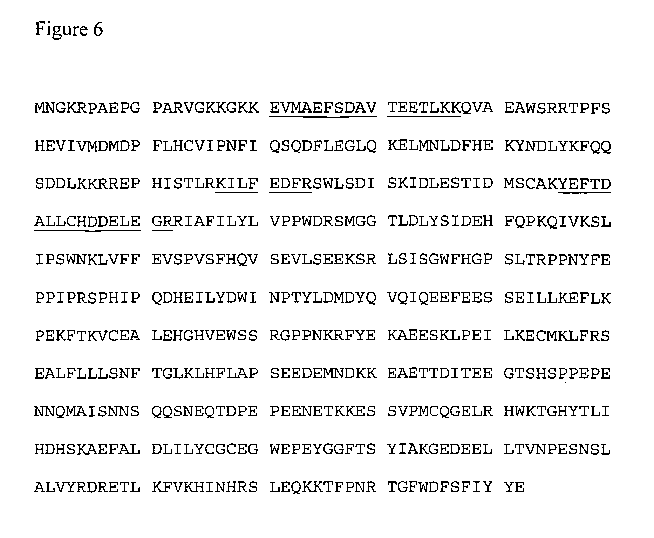

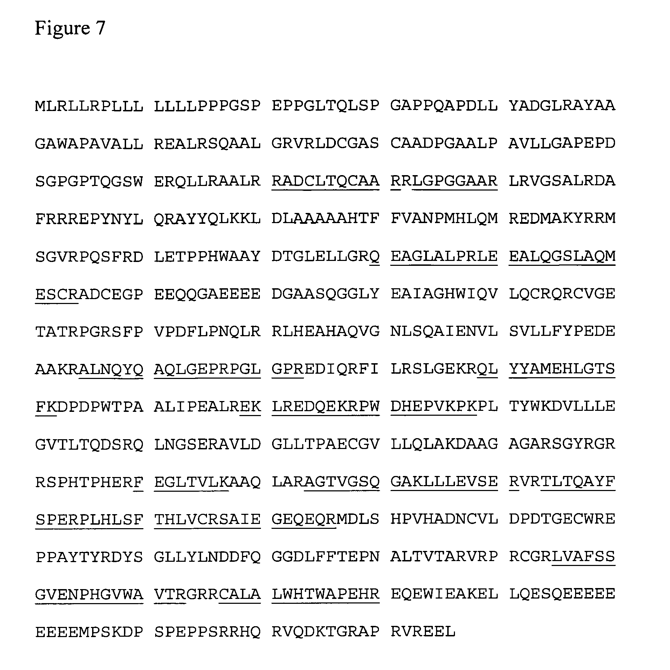

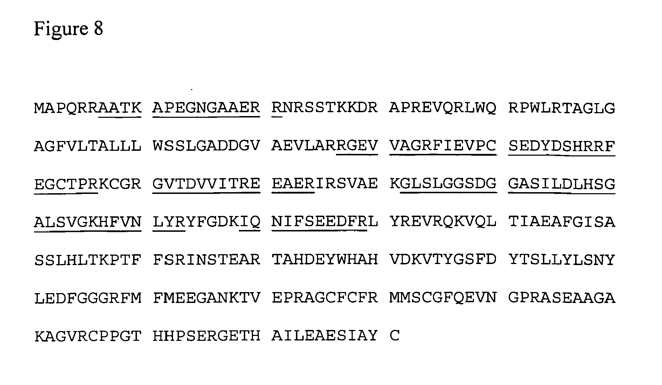

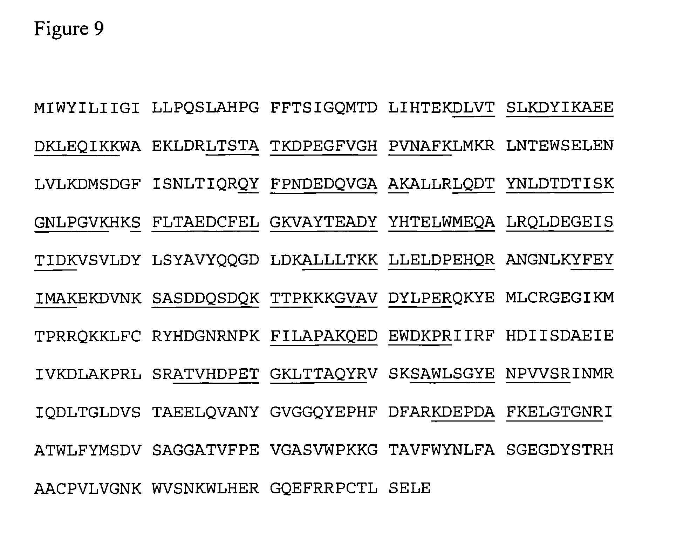

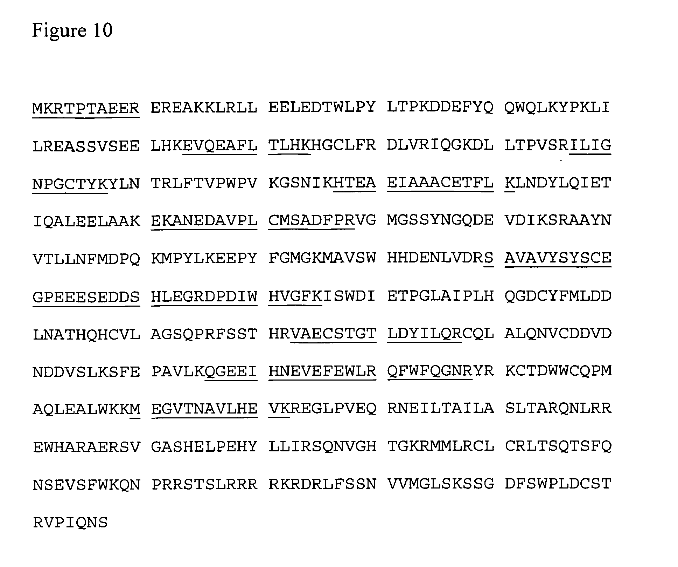

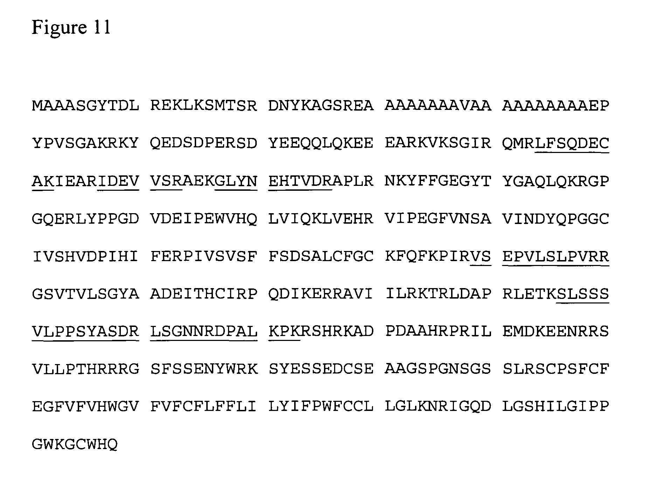

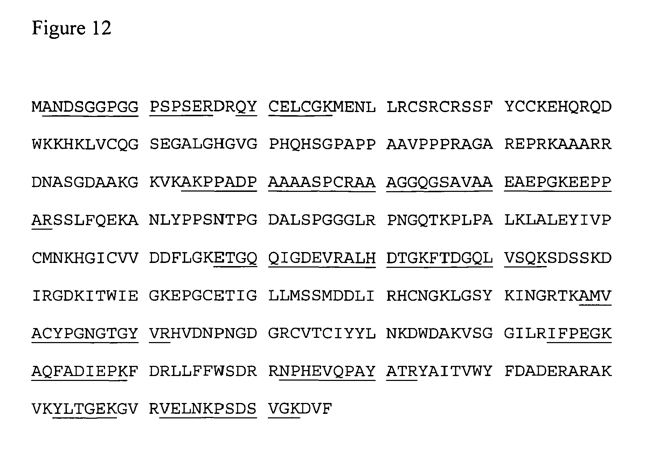

The identified dioxygenases are shown in Table 3 including the competition values for the sample to which 200 µM free compound had been added (fold change relative to DMSO control). Twelve different dioxygenases were identified. For illustration, the identified peptides for individual proteins are shown in Figures 1 to 12. Sequence identifiers are defined by the International Protein Index (IPI) (Kersey et al., 2004. Proteomics 4(7): 1985-1988).

### 1. Cell culture

Human HL-60 cells (DSMZ, Braunschweig, Germany; DSMZ number ACC3) were either obtained from an external supplier (CIL SA, Mons, Belgium) or grown in one litre Spinner flasks (Integra Biosciences, #182101) in suspension in RPMI 1640 medium (Invitrogen, #21875-034) supplemented with 10% Fetal Bovine Serum (Invitrogen, #10270-106) at a density between 0.2 x 10⁶ and 1.0 x 10⁶ cells/ml. Cells were harvested by centrifugation, washed once with 1 x PBS buffer (Invitrogen, #14190-094) and cell pellets were frozen in liquid nitrogen and subsequently stored at -80°C.

### 2. Preparation of cell lysates

Cells were homogenized in a Potter S homogenizer in lysis buffer: 50 mM Tris-HCl, 0.8% NP40, 5% glycerol, 150 mM NaCl, 1.5 mM MgCl₂, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 20 times using a mechanized POTTER S, transferred to 50 ml falcon tubes, incubated for 30 minutes rotating at 4° C and spun down for 10 minutes at 20,000 x g at 4°C (10,000 rpm in Sorvall SLA600, precooled). The supernatant was transferred to an ultracentrifuge (UZ)-polycarbonate tube (Beckmann, 355654) and spun for 1 hour at 145.000 x g at 4°C (40.000 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C.

Extracts of nuclei from the human HL-60 cell line (DSMZ, Braunschweig, Germany; DSMZ number ACC3) were prepared according to the the Dignam protocol (Dignam et al., 1983. Nucleic Acids Res. 11(5):1475-89).

### 3. Capturing of dioxygenases from cell lysate

Sepharose-beads with the immobilized compound (100 µl beads per pull-down experiment) were equilibrated in lysis buffer and incubated with a cell lysate sample containing 50 mg of protein on an end-over-end shaker (Roto Shake Genie, Scientific Industries Inc.) for 2 hours at 4°C. Beads were collected, transferred to Mobicol-columns (MoBiTech 10055) and washed with 10 ml lysis buffer containing 0.4% NP40 detergent, followed by 5 ml lysis buffer containing 0.2 % detergent. To elute bound proteins, 60 µl 2x SDS sample buffer was added to the column. The column was incubated for 30 minutes at 50°C and the eluate was transferred to a siliconized microfuge tube by centrifugation. Proteins were then alkylated with 108 mM iodoacetamid. Proteins were then separated by SDS-Polyacrylamide electrophoresis (SDS-PAGE).

### 4. Protein Identification by Mass Spectrometry

### 4.1 Protein digestion prior to mass spectrometric analysis

Gel-separated proteins were digested in-gel essentially following a previously described procedure (Shevchenko et al., 1996, Anal. Chem. 68:850-858). Briefly, gel-separated proteins were excised from the gel using a clean scalpel, destained twice using 100 µl 5mM triethylammonium bicarbonate buffer (TEAB; Sigma T7408) and 40% ethanol in water and dehydrated with absolute ethanol. Proteins were subsequently digested in-gel with porcine trypsin (Promega) at a protease concentration of 10 ng/µl in 5mM TEAB. Digestion was allowed to proceed for 4 hours at 37°C and the reaction was subsequently stopped using 5 µl 5% formic acid.

### 4.2 Sample preparation prior to analysis by mass spectrometry

Gel plugs were extracted twice with 20 µl 1% formic acid and three times with increasing concentrations of acetonitrile. Extracts were subsequently pooled with acidified digest supernatants and dried in a vacuum centrifuge.

### 4.3 iTRAQ labeling of peptide extracts

The peptide extracts of samples treated with 200 µM of free compound 6 and the solvent control (0.5% DMSO) were treated with different variants of the isobaric tagging reagent (iTRAQ Reagents Multiplex Kit, part number 4352135, Applied Biosystems, Foster City, CA, USA). The iTRAQ reagents are a set of multiplexed, amine-specific, stable isotope reagents that can label peptides on amino groups in up to four different biological samples enabling simultaneous identification and quantitation of peptides. The iTRAQ reagents were used according to instructions provided by the manufacturer. The samples were resuspended in 10 µl 50 mM TEAB solution, pH 8.5 and 10 µl ethanol were added. The iTRAQ reagent was dissolved in 120 µl ethanol and 10 µl of reagent solution were added to the sample. The labeling reaction was performed at room temperature for one hour on a horizontal shaker and stopped by adding 5 µl of 100 mM TEAB and 100 mM glycine in water. The two labeled sampled were then combined, dried in a vacuum centrifuge and resuspended in 10 µl of 0.1% formic acid in water.

### 4.4 Mass spectrometric data acquisition

Peptide samples were injected into a nano LC system (CapLC, Waters or nano-LC 1D+, Eksigent) which was directly coupled either to a quadrupole TOF (QTOF Ultima, QTOF Micro, Waters), ion trap (LTQ) or Orbitrap mass spectrometer. Peptides were separated on the LC system using a gradient of aqueous and organic solvents (see below). Solvent A was 0.1 % formic acid and solvent B was 70% acetonitrile in 0.1 % formic acid.

**Table 2: Peptides elution off the LC system**

| Time (min) | % solvent A | % solvent B |
|---|---|---|
| 0 | 95 | 5 |
| 8.0 | 95 | 5 |
| 15 | 85 | 15 |
| 64.5 | 60 | 40 |
| 84.5 | 38 | 62 |
| 87 | 5 | 95 |
| 91 | 250 | 95 |
| 91.5 | 2095 | 5 |

### 4.5 Protein identification and quantitation

The peptide mass and fragmentation data generated in the LC-MS/MS experiments were used to query a protein data base consisting of an in-house curated version of the International Protein Index (IPI) protein sequence database combined with a decoy version of this database (Elias and Gygi, 2007. Target-decoy search strategy for increased confidence in large-scale protein identifications by mass spectrometry. Nature Methods 4, 207-214). Proteins were identified by correlating the measured peptide mass and fragmentation data with data computed from the entries in the database using the software tool Mascot (Perkins et al., 1999. Probability-based protein identification by searching sequence databases using mass spectrometry data. Electrophoresis 20, 3551-3567). Search criteria varied depending on which mass spectrometer was used for the analysis. Protein acceptance thresholds were adjusted to achieve a false discovery rate of below 1% as suggested by hit rates on the decoy data base (Elias and Gygi, 2007. Target-decoysearch strategy for increased confidence in large-scale protein identifications by mass spectrometry. Nature Methods 4, 207-214). Relative protein quantitation was performed using peak areas of iTRAQ reporter ion signals essentially as described in an earlier publication (Bantscheff et al., 2007. Nature Biotechnology 25, 1035-1044).

**Table 3: Identified dioxygenases with compound A from HL-60 nuclear extract**

| **Representative Sequence** | **Protein name** | **Protein subclass** | **Target class** | **Protein domain** | **Quantified spectra** | **Fold change versus DMSO control** | **DMSO control** |
|---|---|---|---|---|---|---|---|
| IPI00027192.6 | PLOD1 | P4Hc | Fe20G | P4Hc, 20G-Fell_Oxy | 7 | 0,77 | 1 |
| IPI00030255.1 | PLOD3 | P4Hc | Fe20G | P4Hc | 6 | 1,04 | 1 |
| IPI00045839.3 | LEPRE1 | P4Hc | Fe20G | P4Hc | 21 | 0,7 | 1 |
| IPI00055405.3 | ALKBH2 | ALKB | Fe20G | | 26 | 1,21 | 1 |
| IPI00076597.3 | ALKBH3 | ALKB | Fe2OG | 20G-Fell_Oxy | 24 | 0,45 | 1 |
| IPI00170429.3 | OGFOD1 | P4Hc | Fe2OG | P4Hc | 3 | 0,22 | 1 |
| IPI00217056.2 | LEPREL2 | P4Hc | Fe2OG | P4Hc | 24 | 0,21 | 1 |
| IPI00217869.4 | C170RF101 | P4Hc | Fe2OG | P4Hc | 20 | 0,42 | 1 |
| IPI00218682.1 | P4HA1 | P4Hc | Fe20G | P4Hc | 41 | 0,14 | 1 |
| IPI00641635.1 | FTO | ALKB | Fe20G | | 14 | 0,25 | 1 |
| IPI00759562.1 | ALKBH5 | ALKB | Fe2OG | | 7 | 0,48 | 1 |
| IPI00004928.1 | EGLN1 | P4Hc | Fe2OG | P4Hc | 17 | 0,2 | 1 |

### Example 3: Bead assay using immobilized compound A and K562, HL-60 and Jurkat cell lysates

This example demonstrates the use of immobilized compound A for the capturing and identification of dioxygenases from nuclear lysates of three different cell lines. Compound A was added at 100 µM to samples of K-562, HL-60 and Jurkat nuclear lysates thereby allowing the test compound to bind to the target proteins in the lysate. Then the lysate was contacted with the immobilized compound A to capture remaining free target proteins. The proteins bound to the immobilized compound were eluted with detergent-containing buffer and separated on a SDS-polyacryamide gel. The peptide extracts corresponding to samples treated with 100 µM of free compound A or the solvent control (0.5% DMSO) were treated with different variants of the isobaric tagging reagents (Table 4) and analysed by mass spectrometry.

**Table 4: Compound treatment and TMT-labeling of cell samples**

| **Immobilized compound** | **Cell line** | **Free compound** | **TMT label** |
|---|---|---|---|
| Compound A | K-562 | 100µM Compound A | 126 |
| Compound A | K-562 | DMSO | 127 |
| Compound A | HL-60 | 100µM Compound A | 128 |
| Compound A | HL-60 | DMSO | 129 |
| Compound A | Jurkat | 100µM Compound A | 130 |
| Compound A | Jurkat | DMSO | 131 |

### Methods

Jurkat cells (ATCC number TIB-152) were either obtained from an external supplier (CIL SA, Mons, Belgium) or grown in one litre Spinner flasks (Integra Biosciences, #182101) in suspension in RPMI 1640 medium (Invitrogen, #21875-034) supplemented with 10% Fetal Bovine Serum (Invitrogen, #10270-106) at a density between 0.2 x 10⁶ and 1.0 x 10⁶ cells/ml. Cells were harvested by centrifugation, washed once with 1 x PBS buffer (Invitrogen, #14190-094) and cell pellets were frozen in liquid nitrogen and subsequently stored at -80°C.

Source of HL-60 cells: HL-60 cell line (DSMZ, Braunschweig, Germany; DSMZ number ACC3). K562 cells were also purchased from DSMZ (Braunschweig, Germany).

Extracts of nuclei were prepared according to the the Dignam protocol (Dignam et al., 1983. Nucleic Acids Res. 11 (5):1475-89).

### TMT labeling of peptide extracts

The peptide extracts of samples treated with 100 µM of free compound A or DMSO (Table 4) were treated with different variants of the isobaric tagging reagent (TMT sixplex Label Reagent Set, part number 90066, Thermo Fisher Scientific Inc., Rockford, IL 61105 USA). The TMT reagents are a set of multiplexed, amine-specific, stable isotope reagents that can label peptides on amino groups in up to six different biological samples enabling simultaneous identification and quantitation of peptides. The TMT reagents were used according to instructions provided by the manufacturer. The samples were resuspended in 10 µl 50 mM TEAB solution, pH 8.5 and 10 µl acetonitril were added. The TMT reagent was dissolved in acetonitrile to a final concentration of 24 mM and 10 µl of reagent solution were added to the sample. The labeling reaction was performed at room temperature for one hour on a horizontal shaker and stopped by adding 5 µl of 100 mM TEAB and 100 mM glycine in water. The labeled samples were then combined, dried in a vacuum centrifuge and resuspended in 60% 200mM TEAB / 40% acetonitril. 2 µl of a 2.5% NH₂OH solution in water were added, incubated for 15 min and finally the reaction was stopped by addition of 10 µl of 20% formic acid in water. After freeze-drying samples were resuspended in 50 µl 0.1 % formic acid in water.

### Mass spectrometric data acquisition

Peptide samples were injected into a nano LC system (CapLC, Waters or nano-LC 1D+, Eksigent) which was directly coupled either to a quadrupole TOF (QTOF Ultima, QTOF Micro, Waters), ion trap (LTQ) or Orbitrap mass spectrometer. Peptides were separated on the LC system using a gradient of aqueous and organic solvents (see below). Solvent A was 0.1% formic acid and solvent B was 70% acetonitrile in 0.1 % formic acid.

**Table 5: Peptide elution off the LC system**

| Method file | Flow rate (nL/min) | Gradient Time (min)-%B |
|---|---|---|
| PQD_265min | 190 | 00-5.263 |
| | | 07-10 |
| | | 190-40.263 |
| | | 210-52.105 |
| | | 223-60 |
| | | 230-90 |
| | | 236-90 |
| | | 240-5.263 |
| | | 260-5.263 |

### Protein identification and quantitation

The peptide mass and fragmentation data generated in the LC-MS/MS experiments were used to query a protein data base consisting of an in-house curated version of the International Protein Index (IPI) protein sequence database combined with a decoy version of this database (Elias and Gygi, 2007, Target-decoy search strategy for increased confidence in large-scale protein identifications by mass spectrometry. Nature Methods 4, 207-214). Proteins were identified by correlating the measured peptide mass and fragmentation data with data computed from the entries in the database using the software tool Mascot (Perkins et al., 1999. Probability-based protein identification by searching sequence databases using mass spectrometry data. Electrophoresis 20, 3551-3567). Search criteria varied depending on which mass spectrometer was used for the analysis. Protein acceptance thresholds were adjusted to achieve a false discovery rate of below 1% as suggested by hit rates on the decoy data base (Elias and Gygi, 2007. Target-decoy search strategy for increased confidence in large-scale protein identifications by mass spectrometry. Nature Methods 4, 207-214). Relative protein quantitation was performed using peak areas of iTMT reporter ion signals essentially as described in an earlier publication (Bantscheff et al., 2007. Nature Biotechnology 25, 1035-1044).

### Results

The identified dioxygenases are shown in Table 6 including the competition values for the sample to which 100 µM free compound had been added (fold change relative to DMSO control). Seventeen different dioxygenases were identified. Sequence identifiers are defined by the International Protein Index (IPI) (Kersey et al., 2004. Proteomics 4(7):1985-1988).

**Table 6: Identified dioxygenases with compound A from three cell lines**

| | | | | **K-562** | | **HL-60** | | **Jurkat** | |
|---|---|---|---|---|---|---|---|---|---|
| **Representative Sequence** | **Protein name** | **Protein subclass** | **Protein Domain** | **Fold change versus DMSO control** | **DMSO control** | **Fold change versus DMSO control** | **DMSO control** | **Fold change versus DMSO control** | **DMSO control** |
| IPI00003128.1 | P4HA2 | P4Hc | P4Hc | 0,28 | 1 | 2,79 | 1 | 1,36 | 1 |
| IPI00004928.1 | EGLN1 | P4Hc | P4Hc | 0,21 | 1 | 0,24 | 1 | 0,13 | 1 |
| IPI00027192.6 | PLOD1 | P4Hc | P4Hc, 20G-Fell Oxy | 0,35 | 1 | 0,78 | 1 | 0,91 | 1 |
| IPI00030255.1 | PLOD3 | P4Hc | P4Hc | 0,52 | 1 | 1,12 | 1 | 1,61 | 1 |
| IPI00031118.3 | ALKBH7 | ALKB | | 0,54 | 1 | 0,82 | 1 | 0,55 | 1 |
| IPI00045839.3 | LEPRE1 | P4Hc | P4Hc | 1 | 1 | 1,26 | 1 | 0,9 | 1 |
| IPI00055405.3 | ALKBH2 | ALKB | | 0,85 | 1 | 1,78 | 1 | 1,08 | 1 |
| IPI00074957.1 | EGLN2 | P4Hc | P4Hc | 0,75 | 1 | 1,24 | 1 | 1,23 | 1 |
| IPI00076597.3 | ALKBH3 | ALKB | 2OG-Fell Oxy | 0,43 | 1 | 0,36 | 1 | 0,32 | 1 |
| IPI00170429.3 | OGFOD1 | P4Hc | P4Hc | 0,19 | 1 | 0,5 | 1 | 0,3 | 1 |
| IPI00217056.2 | LEPREL2 | P4Hc | P4Hc | 0,25 | 1 | 0,2 | 1 | 0,09 | 1 |
| IPI00217869.4 | C17ORF101 | P4Hc | P4Hc | 0,42 | 1 | 0,67 | 1 | 0,41 | 1 |
| IPI00218682.1 | P4HA1 | P4Hc | P4Hc | 0,31 | 1 | 0,25 | 1 | 0,14 | 1 |
| IPI00301224.2 | TMLHE | TAUD | | 0,5 | 1 | 1,04 | 1 | 0,49 | 1 |
| IPI00337495.3 | PLOD2 | P4Hc | P4Hc | 0,54 | 1 | 0,86 | 1 | 1,09 | 1 |
| IP100641635.1 | FTO | ALKB | | 0,19 | 1 | 0,26 | 1 | 0,08 | 1 |
| IPI00759562.1 | ALKBH5 | ALKB | | 0,44 | 1 | 0,78 | 1 | 0,56 | 1 |

### Example 4: Bead assay using immobilized compound B and HL-60 nuclear extract

This example demonstrates the use of immobilized compound B for the capturing and identification of dioxygenases from cell lysate in a competition binding assay. To the first aliquot of cell lysate 200 µM of the free compound B was added and allowed to bind to proteins in the lysate. Then the affinity matrix with the immobilized compound B was added to capture proteins that were not interacting with the previously added free compound. Beads were separated from the lysate and bead bound proteins were eluted in SDS sample buffer and subsequently separated by SDS-Polyacrylamide gel electrophoresis. Suitable gel bands were cut out and subjected to in-gel proteolytic digestion with trypsin. The second lysate aliquot was processed identically, however no free compound was added (DMSO solvent control). Peptides originating from samples 1 and 2 were labeled with iTRAQ reagents (iTRAQ 115 and iTRAQ 117) and the combined samples were analyzed with a nano-flow liquid chromatography system coupled online to a tandem mass spectrometer (LC-MS/MS) experiment followed by iTRAQ reporter ion quantification in the MS/MS spectra (Ross et al., 2004. Mol. Cell. Proteomics 3(12):1154-1169). Further experimental protocols can be found in WO2006/134056 and a previous publication (Bantscheff et al., 2007. Nature Biotechnology 25, 1035-1044).

The preparation of HL-60 nuclear lysates, capturing of dioxygenases from cell lysate and the protein identification by Mass Spectrometry was performed as described in example 2.

The identified dioxygenases are shown in Table 7 including the competition values for the sample to which 200 µM free compound had been added (fold change relative to DMSO control). Seven different dioxygenases were identified. Sequence identifiers are defined by the International Protein Index (IPI) (Kersey et al., 2004. Proteomics 4(7): 1985-1988).

**Table 7: Identified dioxygenases with compound B from HL-60 nuclear extract**

| **Representative Sequence** | **Protein name** | **Protein subclass** | **Target class** | **Protein Domain** | **Quantified Spectra** | **Fold change versus DMSO control** | **DMSO control** |
|---|---|---|---|---|---|---|---|
| IPI00045839.3 | LEPRE1 | LEPRE1 | Fe2OG | P4Hc | 6 | 0,67 | 1 |
| IPI00076597.3 | ALKBH3 | ALKBH3 | Fe2OG | 2OG-Fell-Oxy | 10 | 0,56 | 1 |
| IPI00217056.2 | LEPREL2 | LEPREL2 | Fe2OG | P4Hc | 19 | 0,13 | 1 |
| IPI00217869.4 | C17ORF101 | C17ORF101 | Fe2OG | P4Hc | 8 | 0,52 | 1 |
| IPI00218682.1 | P4HA1 | P4HA1[1], P4HA1[2] | Fe2OG | P4Hc | 15 | 0,24 | 1 |
| IPI00002458.3 | OGFOD2 | OGFOD2 | Fe2OG | P4Hc | 14 | 0,18 | 1 |
| IPI00004928.1 | EGLN1 | EGLN1 | Fe2OG | P4Hc | 4 | 0,52 | 1 |

### Example 5: Bead assay using immobilized compound C and HL-60 nuclear extract

This example demonstrates the use of immobilized compound C for the capturing and identification of dioxygenases from cell lysate in a competition binding assay. To the first aliquot of cell lysate 200 µM of the free compound C was added and allowed to bind to proteins in the lysate. Then the affinity matrix with the immobilized compound C was added to capture proteins that were not interacting with the previously added free compound. Beads were separated from the lysate and bead bound proteins were eluted in SDS sample buffer and subsequently separated by SDS-Polyacrylamide gel electrophoresis. Suitable gel bands were cut out and subjected to in-gel proteolytic digestion with trypsin. The second lysate aliquot was processed identically, however no free compound was added (DMSO solvent control). Peptides originating from samples 1 and 2 were labeled with iTRAQ reagents (iTRAQ 114 and iTRAQ 116) and the combined samples were analyzed with a nano-flow liquid chromatography system coupled online to a tandem mass spectrometer (LC-MS/MS) experiment followed by iTRAQ reporter ion quantification in the MS/MS spectra (Ross et al., 2004. Mol. Cell. Proteomics 3(12):11154-1169). Further experimental protocols can be found in WO2006/134056 and a previous publication (Bantscheff et al., 2007. Nature Biotechnology 25, 1035-1044).

The preparation of HL-60 nuclear lysates, capturing of dioxygenases from cell lysate and the protein identification by Mass Spectrometry was performed as described in example 2.

The identified dioxygenases are shown in Table 8 including the competition values for the sample to which 200 µM free compound had been added (fold change relative to DMSO control). Seven different dioxygenases were identified. Sequence identifiers are defined by the International Protein Index (IPI) (Kersey et al., 2004. Proteomics 4(7): 1985-1988).

**Table 8: Identified dioxygenases with compound C from HL-60 nuclear extract**

| **Representative sequence** | **Protein name** | **Protein subclass** | **Target class** | **Protein domain** | **Quantified spectra** | **Fold change versus DMSO control** | **DMSO control** |
|---|---|---|---|---|---|---|---|
| IPI00027192.6 | PLOD1 | P4Hc | Fe2OG | P4Hc, 2OG-Fell_Ox v | 2 | 0,51 | 1 |
| IPI00045839.3 | LEPRE1 | P4Hc | Fe2OG | P4Hc | 50 | 0,48 | 1 |
| IPI00076597.3 | ALKBH3 | ALKB | Fe2OG | 2OG- | 7 | 0,45 | 1 |
| IPI00217056.2 | LEPREL2 | P4Hc | Fe2OG | P4Hc | 17 | 0,12 | 1 |
| IPI00217869.4 | C17ORF101 | P4Hc | Fe2OG | P4Hc | 13 | 0,24 | 1 |
| IPI00218682.1 | P4HA1 | P4Hc | Fe2OG | P4Hc | 35 | 0,1 | 1 |
| IPI00004928.1 | EGLN1 | P4Hc | Fe2OG | P4Hc | 34 | 0,19 | 1 |

## Claims

1. An immobilization compound according to formula (I) 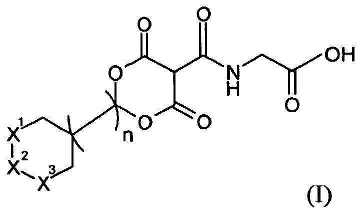 wherein each of X¹, X² , and X³ is CH₂ or NH,
with the proviso that the piperidinyl ring has only one NH-group as a ring member,
and wherein
n is 0 or 1;
or a salt thereof.

2. An immobilization compound selected from the group consisting of formula (II) to formula (IV) 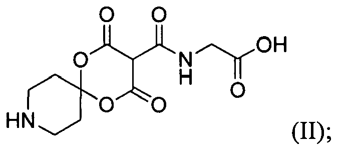 and 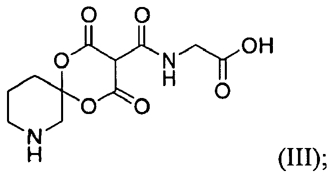 and 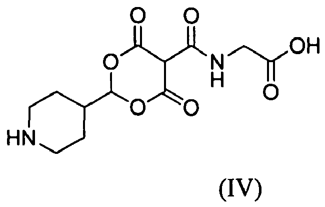 or a salt thereof.

3. A method for the preparation of an immobilization product, wherein the immobilization compound according to formula (I), preferably the immobilization compound selected from the group consisting of formula (II) to formula (IV), is immobilized on a solid support, in particular wherein the solid support is selected from the group consisting of agarose, modified agarose, sepharose beads (e.g. NHS-activated sepharose), latex, cellulose, and ferro- or ferrimagnetic particles.

4. The method of claim 3, wherein the immobilization product results from a covalent direct or linker mediated attachment of the immobilization compound to the solid support, in particular wherein the linker is a C₁₋₁₀ alkylene group, which is optionally interrupted or terminated by one or more atoms or functional groups selected from the group consisting of S, O, NH, C(O)O, OC(O), C(O), NHC(O), and C(O)NH, and wherein the linker is optionally substituted with one or more substituents independently selected from the group consisting of halogen, OH, NH₂, C(O)H, C(O)NH₂, SO₃H, NO₂, and CN.

5. An immobilization product, obtainable by the method of any of claims 3 or 4.

6. An immobilization product, comprising the immobilization compound according to formula (I), preferably the immobilization compound selected from the group consisting of formula (II) to (IV), immobilized on a solid support, in particular wherein the solid support is selected from the group consisting of agarose, modified agarose, sepharose beads (e.g. NHS-activated sepharose), latex, cellulose, and ferro- or ferrimagnetic particles.

7. A method for the identification of a dioxygenase interacting compound, comprising the steps of
a) providing a protein preparation containing at least one dioxygenase,
b) contacting the protein preparation with the immobilization product of any of claims 5 or 6 and with a given compound under conditions allowing the formation of one or more different complexes between one of the dioxygenases and the immobilization product, and
c) detecting the complex or the complexes formed in step b).

8. A method for the identification of a dioxygenase interacting compound, comprising the steps of:
a) providing two aliquots of a protein preparation containing at least one dioxygenase,
b) contacting one aliquot with the immobilization product of any of claims 5 or 6 under conditions allowing the formation of one or more different complexes between one of the dioxygenases and the immobilization product,
c) contacting the other aliquot with the immobilization product of any of claims 5 or 6 and with a given compound under conditions allowing the formation of one or more different complexes between one of the dioxygenases and the immobilization product, and
d) determining the amount of the complex or the complexes formed in steps b) and c).

9. A method for the identification of a dioxygenase interacting compound, comprising the steps of:
a) providing two aliquots of a cell preparation comprising each at least one cell containing at least one dioxygenase,
b) incubating one aliquot with a given compound,
c) harvesting the cells of each aliquot,
d) lysing the cells in order to obtain protein preparations,
e) contacting the protein preparations with the immobilization product of any of claims 5 or 6 under conditions allowing the formation of one or more different complexes between one of the dioxygenases and the immobilization product, and
f) determining the amount of the complex or the complexes formed in each aliquot in step e).

10. The method of any of claims 7 to 9, wherein a reduced amount of the complex formed in the aliquot incubated with the compound in comparison to the aliquot not incubated with the compound indicates that said dioxygenase is a target of the compound.

11. The method of any of claims 7 to 10, wherein the amount of the complex is determined by separating the dioxygenase from the immobilization product and subsequent detection of the separated dioxygenase or subsequent determination of the amount of the separated dioxygenase, in particular wherein the dioxygenase is detected or the amount of the dioxygenase is determined by mass spectrometry or immunodetection methods, preferably with an antibody directed against the dioxygenase.

12. The method of any of claims 7 to 9, wherein said given compound is selected from the group consisting of synthetic compounds, or organic synthetic drugs, more preferably small molecule organic drugs, and natural small molecule compounds.

13. The method of any of claims 7 to 12, wherein the given compound is a dioxygenase inhibitor.

14. The method of any of claims 7 to 12, wherein the provision of a protein preparation includes the steps of harvesting at least one cell containing dioxygenases and lysing the cell.

15. The method of any of claims 7 to 14, wherein the steps of the formation of the complex are performed under essentially physiological conditions.

16. A method for determining the presence of one or more dioxygenases in a sample, comprising the steps of:
a) providing a protein preparation expected to contain said one or more dioxygenase,
b) contacting the protein preparation with the immobilization product of any of claims 5 or 6 under conditions allowing the formation of a complex between one of the dioxygenases and the immobilization product, and
c) detecting whether one or more dioxygenases have formed a complex with the immobilization product.

17. Use of the immobilization compound according to formula (I) or of the immobilization product of any of claims 5 or 6 for the identification of dioxygenase interacting compounds or for the purification of dioxygenases.
